# EUROPEAN PATENT SPECIFICATION

(11) **EP 2 253 287 B1**
(45) Date of publication and mention of the grant of the patent: **15.08.2018**
(21) Application number: 10162897.2
(22) Date of filing: 14.05.2010
(51) Int. Cl.: A61B 90/00, A61B 34/20

(54) **AUTOMATIC REGISTRATION TECHNIQUE**
AUTOMATISCHE REGISTRIERTECHNIK
TECHNIQUE D'ENREGISTREMENT AUTOMATIQUE

(30) Priority: 14.05.2009 US 178437 P
(43) Date of publication of application: 24.11.2010
(73) Proprietor: Covidien LP, Mansfield, MA 02048 (US)
(72) Inventor: Averbuch, Dorian, 47261, Ramat HaSharon (IL)
(74) Representative: Maschio, Antonio

(56) References cited:
- EP-A1- 1 504 713
- EP-A2- 1 717 758
- EP-A2- 1 929 956
- WO-A1-2009/023801
- WO-A2-2008/111070
- JP-A- 2007 125 179
- US-A1- 2001 031 920
- US-A1- 2002 049 375
- US-A1- 2005 107 679
- US-A1- 2006 058 647
- US-A1- 2006 178 828
- US-A1- 2008 118 135
- US-B1- 7 420 555
- KIRALY A P ET AL: "Three-dimensional human airway segmentation methods for clinical virtual bronchoscopy", ACADEMIC RADIOLOGY, RESTON, VA, US, vol. 9, no. 10, 1 September 2002 (2002-09-01), pages 1153-1168, XP002330892, ISSN: 1076-6332, DOI: 10.1016/S1076-6332(03)80517-2

## Description

### BACKGROUND OF THE INVENTION

Breakthrough technology has emerged which allows the navigation of a catheter tip through a tortuous channel, such as those found in the pulmonary system, to a predetermined target. This technology compares the real-time movement of a sensor against a three-dimensional digital map of the targeted area of the body.

Such technology is described in U.S. Patents 6,188,355; 6,226,543; 6,558,333; 6,574,498; 6,593,884; 6,615,155; 6,702,780; 6,711,429; 6,833,814; 6,974,788; and 6,996,430, all to Gilboa or Gilboa et al.; and U.S. Published Applications Pub. Nos. 2002/0193686; 2003/0074011; 2003/0216639; 2004/0249267 to either Gilboa or Gilboa et al.

Using this technology begins with recording a plurality of images of the applicable portion of the patient, for example, the lungs. These images are often recorded using CT technology. CT images are two-dimensional slices of a portion of the patient. After taking several, parallel images, the images may be "assembled" by a computer to form a three-dimensional model, or "CT volume" of the lungs. Typically, so that all of these individual slices can be properly assembled, they are all taken while the patient is at the same point in the breathing cycle, such as maximum inhalation. In other words, the patient is instructed to take a full breath and hold it during the procedure.

The CT volume is used during the procedure as a map to the target. The user navigates a steerable probe that has a trackable sensor at its distal tip. The sensor provides the system with a real-time image of its location. However, because the image of the sensor location appears as a vector on the screen, the image has no context without superimposing the CT volume over the image provided by the sensor. The act of superimposing the CT volume and the sensor image is known as "registration."

There are various registration methods, some of which are described in the aforementioned references, and utilize a probe with a trackable sensor, as described above. For example, point registration involves selecting a plurality of points, typically identifiable anatomical landmarks, inside the lung from the CT volume and then using the sensor (with the help of an endoscope) and "clicking" on each of the corresponding landmarks in the lung. Clicking on the landmarks refers to activating a record feature on the sensor that signifies the registration point should be recorded. The recorded points are then aligned with the points in the CT volume, such that registration is achieved.

This method works well for initial registration in the central area but as the sensor is navigated to the distal portions of the lungs, the registration becomes less accurate as the distal airways are smaller.

Also, the point registration method matches a "snapshot" location of the landmarks to another "snapshot" (CT volume) of the lungs. Each snapshot is taken at different times and, potentially, at different points in the breathing cycle. Due to the dynamic nature of the lungs, the shape of the lungs during the CT scan is likely not the same as the shape of those same lungs during the procedure.

Moreover, because the user is "clicking" on the landmarks over the course of several breathing cycles, it is up to the user to approximate the timing of his clicking so that it roughly matches the point in the breathing cycle at which the CT scan was taken. This leaves much room for error. Finally, it is time consuming for the user to maneuver the sensor tip to the various landmarks.

Another example of a registration method utilizing a trackable sensor involves recording a segment of an airway and shape-match that segment to a corresponding segment in the CT volume.

This method of registration suffers similar setbacks to the point registration method, though it can be used in more distal airways because an endoscope is not required.

The registration should be conducted more than once to keep the registration updated. It may be inconvenient or otherwise undesirable to require additional registration steps from a user.

Additionally, this method requires that a good image exists in the CT volume for any given airway occupied by the sensor. If for example, the CT scan resulted in an airway shadowed by a blood vessel, for example, the registration will suffer because the shape data on that airway is compromised.

Another registration method tailored for trackable sensors is known as "Adaptive Navigation" and was developed and described in U.S. Published Application 2008/0118135 to Averbuch et al.. This registration technique operates on the assumption that the sensor remains in the airways at all times. The position of the sensor is recorded as the sensor is advanced, thus providing a shaped historical path of where the sensor has been. This registration method requires the development of a computer-generated and automatically or manually segmented "Bronchial Tree" (BT). The shape of the historical path is matched to a corresponding shape in the BT.

US2006/0058647 discloses a method of superimposing 3D sensor data of a probe to 2D image data.

EP1504713A1 discloses a navigation system where the superimposed images may be synchronized to various other inputs such as a physiological events.

EP1929956A2 discloses a method for coloring electro-anatomical maps to indicate ultrasound data acquisition, enabling the operator to determine regions where sufficient data have been captured, and guides the operator to areas where additional data collection is still needed.

WO2008/111070A2 discloses co-utilizing intra-procedure imaging together with registered pre-procedure images for performing ongoing corrections to the initial registration of those images.

It would be desirable to develop a registration system that combines some of the advantages of each of the aforementioned registration methods. In particular, it would be desirable to develop a registration method that does not require excess training or skill on the part of the user, allows a fast and accurate initial registration, and integrates seamlessly with the aforementioned adaptive navigation technique once an initial registration has been established.

### DESCRIPTION OF THE INVENTION

The present invention is defined in independent claims 1 and 8 and certain optional features thereof are defined in the dependent claims.

Registration between a digital image of a branched structure and a real-time indicator representing a location of a sensor inside the branched structure is achieved by using the sensor to "paint" a digital picture of the inside of the structure. Once enough location data has been collected, registration is achieved. The registration is "automatic" in the sense that navigation through the branched structure necessarily results in the collection of additional location data and, as a result, registration is continually refined.

In aspects of the invention, methods and systems are provided for registering a real-time position indicator of a sensor on a probe within a branched structure to a three- dimensional model formed from previously-acquired images of said branched structure. In an aspect, a method comprises the following steps and a system is adapted to perform the following steps: moving a probe containing a location sensor within a branched structure; recording data pertaining to locations of said sensor while said sensor is moving through said branched structure; comparing a shape resulting from said data to an interior geometry of passages of said three-dimensional model of said branched structure; and determining a location correlation between said shape and said three-dimensional model based on said comparison. In another aspect, a method comprises the following steps and a system is adapted to perform the following steps: identifying non-tissue space (e.g. air filled cavities) in said three-dimensional model; moving a locatable probe through at least one lumen of said branched structure while recording position data of a location sensor in said probe; and aligning an image representing a location of said probe with an image of said three-dimensional model based on said recorded position data and an assumption that said probe remains located in non-tissue space in said branched structure. The system comprises a control unit that is adapted to perform certain of the method steps. The control unit may be software based and has a processing unit for processing code segments of a software program read from a storage device of the system. The software program is preferably stored on the storage device, typically an electronic non volatile memory chip, an optical storage disc, etc. The software program comprises said code segments for performing said steps of comparing a shape, and determining a location correlation, or aligning an image.

The present invention provides a registration technique useful for establishing at least an initial registration between a three-dimensional operating space and a computer model of that space. The technique is advantageous in that it is fast and easy to establish, and requires no special skill or training on the part of the user navigating a probe or sensor system, such as an endoscope.

For purposes of explanation, the pulmonary airways of the lungs are used herein, though one skilled in the art will realize the embodiments of the system of the present invention could be used in any body cavity or system: circulatory, digestive, pulmonary, to name a few. Additionally, if desired, the embodiments of the system of the present invention may also have non-medical application in navigating in a system using a virtual navigation interface.

The embodiments of the methods that the invention encompasses are also applicable to such body cavities or systems that are reachable without a major surgical intervention being necessary. A major surgical intervention is not included in embodiments of the invention. Surgical interventions that are potentially life threatening are excluded from the methods of embodiments of the invention. It is pointed out that many body cavities are reachable without the need for specifically trained surgeons. The method of embodiments of the invention is limited to use at such body cavities or systems. However, the system adapted for use with such methods may cover broader fields of medical or non-medical applications.

The registration technique, like that of the aforementioned adaptive navigation technique, operates on the premises that (1) the endoscope remains in the airways at all times and (2) recording the movement of a sensor on an endoscope results in a vastly greater sample set than recording discrete positions of a sensor on a stationary endoscope.

The registration method of the present invention may be referred to as "feature-based registration." When the CT scans are taken, the CT machine records each image as a plurality of pixels. When the various scans are assembled together to form a CT volume, voxels (volumetric pixels) appear and can be defined as volume elements, representing values on a regular grid in three dimensional space. Each of the voxels is assigned a number based on the tissue density Hounsfield number. This density value can be associated with gray level or color using well known window-leveling techniques.

The sensing volume of the electromagnetic field of the sensor system is also voxelized by digitizing it into voxels of a specific size compatible with the CT volume. Each voxel visited by the sensor can be assigned a value that correlates to the frequency with which that voxel is visited by the sensor. The densities of the voxels in the CT volume are adjusted according to these values, thereby creating clouds of voxels in the CT volume having varying densities. These voxel clouds or clusters thus match the interior anatomical features of the lungs.

By using a voxel-based approach, registration is actually accomplished by comparing anatomical cavity features to cavity voxels, as opposed to anatomical shapes or locations to structure shapes or locations. An advantage of this approach is that air-filled cavities are of a predictable range of densities. Air filled cavities may be identified as non-tissue space in the CT volume, which is a three-dimensional model. The locatable probe may be moved through the lumen while recording position data thereof. This allows for aligning an image representing a location of said probe with an image of said three-dimensional model based on said recorded position data and an assumption that said probe remains located in non-tissue space. When moving the probe containing a location sensor within a branched structure, data is recorded pertaining to locations of said sensor while said sensor is moving through said branched structure. Then a shape resulting from said data is compared to an interior geometry of passages of said three-dimensional model of said branched structure. This provides for determining a location correlation between said shape and said three-dimensional model based on said comparison.

Registration using the technique of the present invention is accomplished by placing an endoscope or sensor probe into the airways and continually recording its position. Doing so "paints a picture" of the airways that will, depending on the duration of the sample period and the number of airways entered during this exploration phase, fit the three-dimensional model in only one way. The more airways explored, or the more movement in any particular airway, the more accurate the initial registration.

This provides for automatic registration to happen. A navigation system is used to "paint" or draw a three dimensional image of the inside of the airways. This continues until there is enough data for a shape-matching algorithm to determine that the "painted" shape can only fit within the 3D CT volume in one place and orientation.

One particularly elegant feature of the present invention is that quite often, an initial survey of the airways is performed at the beginning of a procedure. If so, the initial survey will typically suffice to establish an initial registration. Hence, from the perspective of the user, a navigation system employing the present invention no longer requires an initial registration phase.

Another way to accomplish initial registration is to simply navigate the probe down a plurality of various airways, preferably selected in both lungs. As stated above, the more airways visited, the smaller the registration error.

Yet another way to accomplish initial registration operates with the understanding that a probe is much smaller than the larger airways, hence a single path through a large airway would result in a large error. However, if a data cloud were obtained by visiting a multitude of points in an airway, especially at the various extents of the airway, registration may be accomplished even though few airways are visited. For example, one probe for use with the present invention includes a curved distal end, others include steerable probes that may be curved at their distal ends. If the probe is inserted into a large airway with the distal end curved, it is possible to rotate the probe around its longitudinal axis while advancing or retracting the probe. Due to the curved distal end of the probe, this rotation combined with the axial movement, will result in a corkscrew that accurately "paints" the walls of the airway. Rotating the probe around its axis is an especially fast way to achieve registration.

One aspect of the present invention provides user feedback during the data collection phase. An indication will appear informing the user that accurate registration has been achieved and that the actual navigation may begin.

This represents a big leap forward in terms of ease-of-use for the users, who used to have to navigate and "mark" a large number of points in the lungs, by "clicking" on each of corresponding landmarks, in order to achieve registration.

Another aspect of the present invention provides user feedback in the form of a registration error indicator. Rather than merely providing a signal indicating that the navigation may commence, an actual registration error may be displayed. This gives the user the option of collecting additional data prior to navigation if increased accuracy is desired. The user also gets the benefit of seeing how his or her actions during the data collection phase affect the accuracy of the registration. Error measurements such as fit, spread and/or tightness may be included. Fit is a term used to describe the overall error of the match. Spread is a term that describes the width of the sample, understanding that widely spaced airway samples will typically result in a better match. Tightness describes how much the sample "cloud" may be moved around within the model airways.

Although the invention has been described in terms of particular embodiments and applications, one of ordinary skill in the art, in light of this teaching, can generate additional embodiments and modifications without exceeding the scope of the appended patent claims. For instance, instead of using an endoscope, other elongate access devices for positioning of the sensors may be used in the system, e.g. a catheter or a needle. Accordingly, it is to be understood that the description herein is proffered by way of example to facilitate comprehension of the invention and should not be construed to limit the scope thereof as defined by the appended patent claims.

## Claims

1. A method of registering a real-time position indicator of a location sensor on a probe within a branched structure to a three-dimensional model represented by voxels formed from previously-acquired images of said branched structure, comprising:
recording data pertaining to locations of said sensor in said branched structure, and
utilizing a processor to
establish a cavity voxel cloud representing a shape of an anatomical cavity of said branched structure based on said data,
compare a shape represented by said cavity voxel cloud and resulting from said data to an interior geometry of passages of said three-dimensional model of said branched structure, and
determine a location correlation between said cavity voxel cloud representing said shape and said three-dimensional model based on said comparison;
or
identifying non-tissue space in said three dimensional model,
recording position data of a location sensor in a locatable probe in a lumen of said branched structure, and
utilizing a processor to
establish a cavity voxel cloud representing a shape of an anatomical cavity of said branched structure based on said data,
compare a shape represented by said cavity voxel cloud and resulting from said data to an interior geometry of passages of said three dimensional model of said branched structure, and
align an image representing a location of said probe with an image of said three-dimensional model based on said position data and an assumption that said probe remains located in non-tissue space in said branched structure.

2. The method of claim 1, wherein recording data pertaining to locations of said sensor in said branched structure comprises obtaining a data cloud; or
wherein recording position data of a location sensor in said probe comprises obtaining a data cloud.

3. The method of claim 1 or 2, wherein said probe has a curved distal end containing a location sensor.

4. The method of any of claims 1 to 3, further comprising providing feedback to a user regarding registration.

5. The method of claim 4, wherein providing feedback to a user regarding registration comprises alerting the user that acceptably accurate registration has been achieved.

6. The method of claim 4, wherein providing feedback to a user regarding registration comprises displaying an actual registration error.

7. The method of claim 4, wherein providing feedback to a user regarding registration comprises providing fit, and/or providing spread, and/ or providing tightness.

8. The method of any preceding claim, wherein the said sensor is of a sensor system which produces an electromagnetic field providing a sensing volume and the sensing volume is voxelised into voxels of a size compatible with the voxels of the three dimensional model.

9. The method of claim 8, wherein each voxel of the sensing volume visited by the sensor is assigned a value that correlates to the frequency with which that voxel is visited by the sensor and the density of voxels in the three dimensional model is adjusted according to the values.

10. A navigation system comprising a probe and a location sensor on said probe, forming a locatable probe adapted to register a real-time position indicator of a said sensor on said probe within a branched structure to a three-dimensional model represented by voxels formed from previously-acquired images of said branched structure,
wherein said system is devised to record data pertaining to locations of said sensor while said sensor is moving through said branched structure, and
wherein said system comprises a control unit adapted to establish a cavity voxel cloud representing a shape of an anatomical cavity of said branched structure based on said data and to compare a shape represented by said cavity voxel cloud and resulting from said data to an interior geometry of passages of said three-dimensional model of said branched structure, and determine a location correlation between said cavity voxel cloud and said three- dimensional model based on said comparison;
wherein said system is configured to identify non-tissue space in said three-dimensional model, and record position data of said location sensor in said probe while said locatable probe is moving through at least one lumen of said branched structure while, and wherein said system comprises a control unit adapted to establish a cavity voxel cloud representing a shape of an anatomical cavity of said branched structure based on said data and to compare a shape represented by said cavity voxel cloud and resulting from said data to an interior geometry of passages of said three-dimensional model of said branched structure, and align an image representing a location of said probe with an image of said three-dimensional model based on said position data and an assumption that said probe remains located in non-tissue space in said branched structure.

11. The system of claim 10, wherein said probe has a curved distal end having arranged said location sensor, and wherein said probe is rotatable around a longitudinal axis of the probe, such that upon rotating the probe around the longitudinal axis while advancing or retracting the probe, a corkscrew movement is achieved that provides location data of interior walls of said branched structure.

12. The system of claim 10 or 11, wherein the said sensor is of a sensor system which produces an electromagnetic field providing a sensing volume and configured such that the sensing volume is voxelised into voxels of a size compatible with the voxels of the three-dimensional model.

13. The system of claim 12, configured such that each voxel of the sensing volume visited by the sensor is assigned a value that correlates to the frequency with which that voxel is visited by the sensor and the density of voxels in the three dimensional model is adjusted according to the values.

14. The system of claim 8 or 9, further comprising a unit adapted to provide feedback to a user regarding registration, such as for alerting the user that acceptably accurate registration has been achieved, or displaying an actual registration error.

15. The system of claim 10, wherein said feedback to said user regarding registration comprises providing fit, and/or providing spread, and/or providing tightness.

16. The system of claim 8, 9, 10or 11,wherein the three dimensional model is a CT model, and the sensor is of an electromagnetic sensing system, the sensing volume of an electromagnetic field of which is voxelised into voxels of a size compatible with the CT model.

17. The method of any one of claims 1 to 7, wherein wherein the three dimensional model is a CT model, and the sensor is of an electromagnetic sensing system, the sensing volume of an electromagnetic field of which is voxelised into voxels of a size compatible with the CT model.

## Patentansprüche

1. Verfahren zum Registrieren eines Echtzeitpositionsanzeigers eines Lokalisierungssensors an einer Sonde innerhalb einer verzweigten Struktur an einem durch Voxel dargestellten dreidimensionalen Modell, gebildet aus zuvor aufgenommenen Bildern der verzweigten Struktur, umfassend:
Erfassen von Daten zu Orten des Sensors in der verzweigten Struktur und
Verwenden eines Prozessors, um
eine Hohlraumvoxelwolke, die eine Form eines anatomischen Hohlraums der verzweigten Struktur darstellt, basierend auf den Daten aufzubauen,
eine Form, die durch die Hohlraumvoxelwolke dargestellt wird und sich aus den Daten ergibt, mit einer Innengeometrie von Durchgängen des dreidimensionalen Modells der verzweigten Struktur zu vergleichen, und
eine Ortskorrelation zwischen der Hohlraumvoxelwolke, die die Form darstellt, und dem dreidimensionalen Modell basierend auf dem Vergleich zu bestimmen;
oder
Identifizieren von Nicht-Gewebe-Raum in dem dreidimensionalen Modell,
Erfassen von Positionsdaten eines Lokalisierungssensors in einer lokalisierbaren Sonde in einem Lumen der verzweigten Struktur und
Verwenden eines Prozessors, um
eine Hohlraumvoxelwolke, die eine Form eines anatomischen Hohlraums der verzweigten Struktur darstellt, basierend auf den Daten aufzubauen,
eine Form, die durch die Hohlraumvoxelwolke dargestellt wird und sich aus den Daten ergibt, mit einer Innengeometrie von Durchgängen des dreidimensionalen Modells der verzweigten Struktur zu vergleichen, und
ein Bild, das einen Ort der Sonde darstellt, mit einem Bild des dreidimensionalen Modells basierend auf den Positionsdaten und einer Annahme, dass die Sonde im Nicht-Gewebe-Raum in der verzweigten Struktur lokalisiert bleibt, auszurichten.

2. Verfahren nach Anspruch 1, wobei Erfassen von Daten zu Orten des Sensors in der verzweigten Struktur Erhalten einer Datenwolke umfasst; oder
wobei Erfassen von Positionsdaten eines Lokalisierungssensors in der Sonde Erhalten einer Datenwolke umfasst.

3. Verfahren nach Anspruch 1 oder 2, wobei die Sonde ein gekrümmtes distales Ende aufweist, das einen Lokalisierungssensor aufweist.

4. Verfahren nach einem der Ansprüche 1 bis 3, weiter umfassend Bereitstellen von Feedback an einen Benutzer bezüglich Registrierung.

5. Verfahren nach Anspruch 4, wobei Bereitstellen von Feedback an einen Benutzer bezüglich Registrierung Benachrichtigen des Benutzers, dass eine annehmbare genaue Registrierung erreicht wurde, umfasst.

6. Verfahren nach Anspruch 4, wobei Bereitstellen von Feedback an einen Benutzer bezüglich Registrierung Anzeigen eines tatsächlichen Registrierfehlers umfasst.

7. Verfahren nach Anspruch 4, wobei Bereitstellen von Feedback an einen Benutzer bezüglich Registrierung Bereitstellen von Passung und/oder Bereitstellen von Ausbreitung und/oder Bereitstellen von Festigkeit umfasst.

8. Verfahren nach einem vorstehenden Anspruch, wobei der Sensor von einem Sensorsystem ist, welches ein elektromagnetisches Feld erzeugt, das ein Sensorvolumen bereitstellt, und das Sensorvolumen in Voxel einer Größe, die mit den Voxeln des dreidimensionalen Modells kompatibel ist, voxelisiert wird.

9. Verfahren nach Anspruch 8, wobei jedem Voxel des von dem Sensor besuchten Sensorvolumens ein Wert zugeordnet wird, der mit der Frequenz korreliert, mit der dieser Voxel durch den Sensor besucht wird, und die Dichte von Voxeln in dem dreidimensionalen Modell nach den Werten eingestellt wird.

10. Navigationssystem umfassend eine Sonde und einen Lokalisierungssensor an der Sonde, der eine lokalisierbare Sonde bildet, die angepasst ist, um einen Echtzeitpositionsanzeiger eines Sensors an der Sonde innerhalb einer verzweigten Struktur an einem durch Voxel dargestellten dreidimensionalen Modell, gebildet aus zuvor aufgenommenen Bildern der verzweigten Struktur, zu registrieren,
wobei das System konzipiert ist, um Daten zu Orten des Sensors zu erfassen, während sich der Sensor durch die verzweigte Struktur bewegt, und
wobei das System eine Steuereinheit umfasst, die angepasst ist, um eine Hohlraumvoxelwolke, die eine Form eines anatomischen Hohlraums der verzweigten Struktur darstellt, basierend auf den Daten aufzubauen und eine Form, die durch die Hohlraumvoxelwolke dargestellt wird und sich aus den Daten ergibt, mit einer Innengeometrie von Durchgängen des dreidimensionalen Modells der verzweigten Struktur zu vergleichen und eine Ortskorrelation zwischen der Hohlraumvoxelwolke und dem dreidimensionalen Modell basierend auf dem Vergleich zu bestimmen;
wobei das System konfiguriert ist, um Nicht-Gewebe-Raum in dem dreidimensionalen Modell zu identifizieren und Positionsdaten des Lokalisierungssensors in der Sonde zu erfassen, während sich die lokalisierbare Sonde durch zumindest ein Lumen der verzweigten Struktur bewegt während, und wobei das System eine Steuereinheit umfasst, die angepasst ist, um eine Hohlraumvoxelwolke, die eine Form eines anatomischen Hohlraums der verzweigten Struktur darstellt, basierend auf den Daten aufzubauen und eine Form, die durch die Hohlraumvoxelwolke dargestellt wird und sich aus den Daten ergibt, mit einer Innengeometrie von Durchgängen des dreidimensionalen Modells der verzweigten Struktur zu vergleichen und ein Bild, das einen Ort der Sonde darstellt, mit einem Bild des dreidimensionalen Modells basierend auf den Positionsdaten und einer Annahme, dass die Sonde im Nicht-Gewebe-Raum in der verzweigten Struktur lokalisiert bleibt, auszurichten.

11. System nach Anspruch 10, wobei die Sonde ein gekrümmtes distales Ende aufweist, das den Lokalisierungssensor angeordnet hat, und wobei die Sonde um eine Längsachse der Sonde rotierbar ist, so dass bei Rotation der Sonde um die Längsachse während einem Vor- oder Zurückziehen der Sonde eine Korkenzieherbewegung erreicht wird, die Ortsdaten von Innenwänden der verzweigten Struktur bereitstellt.

12. System nach Anspruch 10 oder 11, wobei der Sensor von einem Sensorsystem ist, welches ein elektromagnetisches Feld erzeugt, das ein Sensorvolumen bereitstellt und konfiguriert ist, so dass das Sensorvolumen in Voxel einer Größe, die mit den Voxeln des dreidimensionalen Modells kompatibel sind, voxelisiert wird.

13. System nach Anspruch 12, das konfiguriert ist, so dass jedem Voxel des von dem Sensor besuchten Sensorvolumens ein Wert zugeordnet wird, der mit der Frequenz korreliert, mit der dieser Voxel von den Sensor besucht wird, und die Dichte von Voxeln in dem dreidimensionalen Modell nach den Werten eingestellt wird.

14. System nach Anspruch 8 oder 9, weiter umfassend eine Einheit, die angepasst ist, um Feedback an einen Benutzer bezüglich Registrierung bereitzustellen, wie etwa zum Benachrichtigen des Benutzers, dass eine annehmbare genaue Registrierung erreicht wurde, oder Anzeigen eines tatsächlichen Registrierfehlers.

15. System nach Anspruch 10, wobei das Feedback an den Benutzer bezüglich Registrierung Bereitstellen von Passung und/oder Bereitstellen von Ausbreitung und/oder Bereitstellen von Festigkeit umfasst.

16. System nach Anspruch 8, 9, 10 oder 11, wobei das dreidimensionale Modell ein CT-Modell ist und der Sensor von einem elektromagnetischen Sensorsystem ist, wobei das Sensorvolumen eines elektromagnetischen Felds von diesem in Voxel einer Größe, die mit dem CT-Modell kompatibel ist, voxelisiert wird.

17. Verfahren nach einem der Ansprüche 1 bis 7, wobei das dreidimensionale Modell ein CT-Modell ist und der Sensor von einem elektromagnetischen Sensorsystem ist, wobei das Sensorvolumen eines elektromagnetischen Felds von diesem in Voxel einer Größe, die mit dem CT-Modell kompatibel ist, voxelisiert wird.

## Revendications

1. Dispositif d'enregistrement d'un indicateur de position en temps réel d'un détecteur de localisation sur une sonde à l'intérieur d'une structure ramifiée vers un modèle en trois dimensions représenté par des voxels formés à partir d'images préalablement acquises de ladite structure ramifiée, comprenant :
l'enregistrement de données relatives aux localisations dudit détecteur dans ladite structure ramifiée, et
l'utilisation d'un processeur pour
établir un nuage de voxels de cavité représentant une forme d'une cavité anatomique de ladite structure ramifiée sur la base desdites données,
comparer une forme représentée par ledit nuage de voxels de cavité et résultant desdites données à une géométrie intérieure des passages dudit modèle en trois dimensions de ladite structure ramifiée, et
déterminer une corrélation de localisation entre ledit nuage de voxels de cavité représentant ladite forme et ledit modèle en trois dimensions sur la base de ladite comparaison ;
ou
l'identification d'espace non tissulaire dans ledit modèle en trois dimensions,
l'enregistrement de données de position d'un détecteur de localisation dans une sonde localisable dans une lumière de ladite structure ramifiée, et
l'utilisation d'un processeur pour
établir un nuage de voxels de cavité représentant une forme d'une cavité anatomique de ladite structure ramifiée sur la base desdites données,
comparer une forme représentée par ledit nuage de voxels de cavité et résultant desdites données à une géométrie intérieure des passages dudit modèle en trois dimensions de ladite structure ramifiée, et
aligner une image représentant une localisation de ladite sonde avec une image dudit modèle en trois dimensions sur la base desdites données de position et d'une hypothèse selon laquelle la sonde reste localisée dans l'espace non tissulaire dans ladite structure ramifiée.

2. Procédé selon la revendication 1, dans lequel l'enregistrement de données relatives aux localisations dudit détecteur dans ladite structure ramifiée comprend l'obtention d'un nuage de données ; ou
dans lequel l'enregistrement de données de position d'un détecteur de localisation dans ladite sonde comprend l'obtention d'un nuage de données.

3. Procédé selon la revendication 1 ou 2, dans lequel ladite sonde a une extrémité distale incurvée contenant un détecteur de localisation.

4. Procédé selon l'une quelconque des revendications 1 à 3, comprenant en outre la fourniture d'un retour d'information à un utilisateur concernant l'enregistrement.

5. Procédé selon la revendication 4, dans lequel la fourniture d'un retour d'information à un utilisateur concernant l'enregistrement comprend le fait d'alerter l'utilisateur qu'un enregistrement suffisamment précis a été effectué.

6. Procédé selon la revendication 4, dans lequel la fourniture d'un retour d'information à un utilisateur concernant l'enregistrement comprend l'affichage d'une erreur d'enregistrement réelle.

7. Procédé selon la revendication 4, dans lequel la fourniture d'un retour d'information à un utilisateur concernant l'enregistrement comprend la fourniture d'une correspondance, et/ou la fourniture d'une extension, et/ou la fourniture d'un resserrement.

8. Procédé selon l'une quelconque des revendications précédentes, dans lequel ledit détecteur est constitué par un système de détecteurs qui produit un champ électromagnétique fournissant un volume de détection et le volume de détection est voxelisé en des voxels d'une taille compatible avec les voxels du modèle en trois dimensions.

9. Procédé selon la revendication 8, dans lequel chaque voxel du volume de détection visité par le détecteur se voit assigner une valeur qui est corrélée à la fréquence à laquelle ce voxel est visité par le détecteur et la densité des voxels dans le modèle en trois dimensions est ajustée en fonction des valeurs.

10. Système de navigation comprenant une sonde et un détecteur de localisation sur ladite sonde, formant une sonde localisable conçue pour enregistrer un indicateur de position en temps réel dudit détecteur sur ladite sonde à l'intérieur d'une structure ramifiée vers un modèle en trois dimensions représenté par des voxels formés à partir d'images préalablement acquises de ladite structure ramifiée,
dans lequel ledit système est conçu pour enregistrer des données relatives aux localisations dudit détecteur tandis que ledit détecteur se déplace à travers ladite structure ramifiée, et
dans lequel ledit système comprend une unité de commande conçue pour établir un nuage de voxels de cavité représentant une forme d'une cavité anatomique de ladite structure ramifiée sur la base desdites données et pour comparer une forme représentée par ledit nuage de voxels de cavité et résultant desdites données à une géométrie intérieure des passages dudit modèle en trois dimensions de ladite structure ramifiée, et déterminer une corrélation de localisation entre ledit nuage de voxels de cavité et ledit modèle en trois dimensions sur la base de ladite comparaison ;
dans lequel ledit système est configuré pour identifier l'espace non tissulaire dans ledit modèle en trois dimensions, et enregistrer des données de position dudit détecteur de localisation dans ladite sonde tandis que ladite sonde localisable se déplace à travers au moins une lumière de ladite structure ramifiée tandis que, et dans lequel ledit système comprend une unité de commande conçue pour établir un nuage de voxels de cavité représentant une forme d'une cavité anatomique de ladite structure ramifiée sur la base desdites données, et pour comparer une forme représentée par ledit nuage de voxels de cavité et résultant desdites données à une géométrie intérieure des passages dudit modèle en trois dimensions de ladite structure ramifiée, et aligner une image représentant une localisation de ladite sonde avec une image dudit modèle en trois dimensions sur la base desdites données de position et d'une hypothèse selon laquelle ladite sonde reste localisée dans l'espace non tissulaire de ladite structure ramifiée.

11. Système selon la revendication 10, dans lequel ladite sonde a une extrémité distale incurvée ayant agencé ledit détecteur de localisation, et dans lequel ladite sonde peut tourner autour d'un axe longitudinal de la sonde, de telle sorte que lors de la rotation de la sonde autour de l'axe longitudinal en avançant ou en rétractant la sonde, un mouvement de tire-bouchon est effectué qui fournit des données de localisation des parois intérieures de ladite structure ramifiée.

12. Système selon la revendication 10 ou 11, dans lequel ledit détecteur est constitué par un système de détecteurs qui produit un champ électromagnétique fournissant un volume de détection et le volume de détection est voxelisé en des voxels d'une taille compatible avec les voxels du modèle en trois dimensions.

13. Système selon la revendication 12, configuré de sorte que chaque voxel du volume de détection visité par le détecteur se voit assigner une valeur qui est corrélée à la fréquence à laquelle ce voxel est visité par le détecteur et la densité des voxels dans le modèle en trois dimensions est ajustée en fonction des valeurs.

14. Système selon la revendication 8 ou 9, comprenant en outre une unité conçue pour fournir un retour d'information à un utilisateur concernant l'enregistrement, tel que pour alerter l'utilisateur qu'un enregistrement suffisamment précis a été effectué, ou afficher une erreur d'enregistrement réelle.

15. Système selon la revendication 10, dans lequel ledit retour d'information audit utilisateur concernant l'enregistrement comprend la fourniture d'une correspondance, et/ou la fourniture d'une extension, et/ou la fourniture d'un resserrement.

16. Système selon la revendication 8, 9, 10 ou 11, dans lequel le modèle en trois dimensions est un modèle CT, et le détecteur est constitué par un système de détection électromagnétique, dont le volume de détection d'un champ électromagnétique est voxelisé en des voxels d'une taille compatible avec le modèle CT.

17. Procédé selon l'une quelconque des revendications 1 à 7, dans lequel le modèle en trois dimensions est un modèle CT, et le détecteur est constitué par un système de détection électromagnétique, dont le volume de détection d'un champ électromagnétique est voxelisé en des voxels d'une taille compatible avec le modèle CT.
